# EUROPEAN PATENT APPLICATION

(11) **EP 4 245 764 A1**
(43) Date of publication of application: **20.09.2023**
(21) Application number: 22382259.4
(22) Date of filing: 18.03.2022
(51) Int. Cl.: C07H 15/04, A61K 47/56, A61K 47/64, A61P 37/06

(54) **NEW CARBOHYDRATE DERIVATIVES AS MIMETICS OF BLOOD GROUP A AND B ANTIGENS**

(71) Applicant: RemAb Therapeutics SL, 08193 Cerdanyola Del Vallès (ES)
(72) Inventor: BELLO GIL, Daniel, 08193 CERDANYOLA DEL VALLÈS (ES); OLIVERA ARDID, Sara, 08193 CERDANYOLA DEL VALLÈS (ES); FERRERO ALVES, Yara, 08193 CERDANYOLA DEL VALLÈS (ES)
(74) Representative: Sugrañes, S.L.P.

(57) **Abstract**

The present invention relates to new carbohydrate derivatives with activity as mimetics of blood group A and B antigens. The present invention also relates to a method for preparing said carbohydrate derivatives and to a glycoconjugate comprising them bound to a polymeric support. The invention also relates to the use of the glycoconjugates for inhibiting and/or removing anti-A and/or anti-B antibodies from blood and blood derivatives and by- products, and, in particular to their use for avoiding rejection in subjects undergoing in organ transplant or haemolytic reactions in blood transfusions from incompatible donors.

## Description

### Technical Field

The present invention relates to new carbohydrate derivatives with activity as mimetics of blood group A and B antigens.

### Technical Background

The ABO blood classification is used to denote the presence of group A, group B and group O (H) antigens (ABH antigens) on erythrocytes.

Group A and B antigens are carbohydrate molecules comprising a terminal trisaccharide with the characteristic feature that the nonreducing terminal carbohydrate of said trisaccharide is either a *N*-acetylgalactosamine or a galactose residue, respectively, which is linked by an α (1→3) glycosidic bond to another galactose residue, which, in turn, bears a fucose residue linked at the 2 position through a a (1→2) glycosidic linkage.

The ABH antigens can be further divided into subgroups, depending on the inner core saccharide chain, for example, they are expressed on type 1 (Galβ1,3GlcNAc), type 2 (Galβ1,4GlcNAc), type 3 (Galβ1,3GalNAcα) and type 4 (Galβ1,3GalNAcβ) chains, the only difference being the linkage between the inner galactose residue and an additional carbohydrate residue, which is either N-acetylglucosamine or *N*-acetylgalactosamine. Thus, for example, group A type 2 antigen has the sequence GalNAcα1,3(Fucα1,2)Galβ1,4GlcNAc and group B type 2 antigen has the sequence Galα1,3(Fucα1,2)Galβ1,4GlcNAc.

The ABO blood group antigens are located on the surfaces of red blood cells and epithelial cells and they are medically highly relevant, especially in the field of transfusions and transplantation, due to their associated anti-A and anti-B antibodies. As the blood group antigen that is expressed differs between individuals, preformed antibodies against the blood group A and B epitopes are produced in individuals not expressing these antigens and they therefore represent the first obstacle for donation of blood, organs or tissues between individuals who express incompatible blood groups. These natural antibodies mainly belong to the IgM isotype and are carbohydrate-binding proteins (CBPs), which bind to the carbohydrate epitopes at the cell surface, leading to complement fixation, and causing intravascular haemolysis of red blood cells in blood transfusion and hyperacute rejection of vascularised organs.

Due to the chronic shortage of donors, strategies are being developed to perform blood transfusions and organ transplants across the ABO barrier.

One strategy known in the art for allowing ABO-incompatible transplantations is the use of carbohydrate-based therapeutics to eliminate/inhibit the recipient preformed anti-carbohydrate antibodies before grafting and for a defined period post-transplantation, until accommodation occurs. For example, extracorporeal anti-carbohydrate removal can be achieved by using carbohydrate-based immunoadsorbents or, alternatively (or complementarily), the anti-carbohydrate antibodies may be inhibited *in vivo* by injectable antigens, as disclosed, for example, in Holgerson et al., Immunol. Cell Biol., 2005, 83, 694-708.

For example, it has been disclosed in the art the use of blood group A and B trisaccharide epitopes linked to crystalline silica and to Sepharose^{®} for removal of anti-A/B antibodies (Holgerson *et al., op. cit*.)

As for the injection of material to inhibit antibodies *in vivo,* the main difficulties for the use of free oligosaccharides are the low affinity protein-carbohydrate binding and short serum half-life. Therefore, the use of glycoconjugates is advantageous, where multiple antigenic oligosaccharides are bound to a biodegradable backbone, allowing multivalent binding and, therefore, increased affinity and inhibiting activity, as disclosed, for example, in Duthaler et a/., Chimia, 2010, 64, 23-28, or in Katopodis et al., J. Clin. Invest., 2002, 110, 1869-1877, or in Wang et al., J. Am. Chem. Soc., 1999, 121, 8174-8181.

So far, it has been always disclosed in the art that, either for *in vitro,* extracorporeal or for *in vivo* inhibition/adsorption of natural antibodies, it is important to use inhibitors or adsorbents bearing active epitopes that correspond as far as possible to the natural carbohydrate antigens.

Thus, for example, in the European patent EP-B-2010920 it is disclosed the preparation of a solid support, in particular, in the form of microbeads, to which several A and B antigens are attached, and it is also disclosed their use for determining the presence of blood group reactive antibodies in serum samples, or for removing blood group reactive antibodies in serum. The carbohydrate antigens are specifically selected from either type 1, 2, 3 or 4 antigens A or B.

Analogously, in the international patent application WO-A-2013/062479 it is disclosed a method for extracorporeal elimination of anti-A and/or anti-B antibodies from blood with an absorbent substance comprising a matrix linked to a saccharide which is a blood group determinant A or a blood group determinant B, namely, a group A-trisaccharide determinant (GalNAcα1,3(Fucα1,2)Galβ1) or a group B-trisaccharide determinant (Galα1,3(Fucα1,2)Galβ1) further linked to inner saccharide as subtype 1, 2, 3 or 4. The matrix is, for example, agarose, dextran, starch or starch derivatives, amylose, amylopectin or polyacrylamide.

The international patent application WO-A-2018/167230 discloses polymers comprising a multitude of carbohydrate ligands, which bind to different types of carbohydrate-binding proteins, including agglutinins, and their use for diagnosis and treatment of diseases, including those associated with CBP-mediated agglutination. Specific examples 9, 32 and 35 correspond to a polylysine glycoconjugate bearing the blood group A antigen type 5 tetrasaccharide, the blood group A trisaccharide antigen and the blood group B trisaccharide antigen, respectively, wherein such antigens are bound to the polylysine backbone through a linker chain group.

The group A and B glycopolymers disclosed so far in the art, comprising the natural group A and B determinants, have still some drawbacks derived from the low affinity binding between these natural antigens and the antibodies.

Therefore, there is still the need to provide new glycoconjugates having improved affinity towards anti-A and/or anti-B antibodies to be used in different biological and medical applications, for example, for effectively removing/inhibiting those antibodies before ABO-incompatible transplantation.

### Object of the invention

The object of the present invention is a compound of formula (I).

Another aspect of the present invention is a process for the preparation of the compound of formula (I).

Another aspect of the invention is a glycoconjugate of the compound of formula (I).

Another aspect of the invention is the use of said glycoconjugate for inhibiting and/or removing anti-A and/or anti-B antibodies from samples of blood or other blood derivatives and by-products.

Another aspect of the invention is said glycoconjugate for use in medicine.

Another aspect of the invention is a pharmaceutical composition comprising said glycoconjugate and at least one pharmaceutically acceptable excipient.

### Description of the drawings

Figure 1 shows Scheme I, depicting a synthetic route for the compounds of formula (I).
Figure 2 and Figure 3 show Schemes Ia and Ib, respectively, depicting a synthetic route for the compounds of formula (I) wherein R₂ is a group of formula (II) and R₃ is a group of formula (III).
Figure 4 shows Scheme Ic, depicting a synthetic route for the compounds of formula (I) wherein R₂ is OH and R₃ is a group of formula (III).
Figure 5 shows Scheme II, depicting the preparation of a glycoconjugate of the compound of formula (I) with polyacrylic acid.
Figure 6 and Figure 7 show Schemes III and IV, respectively, depicting the preparation of a glycoconjugate of the compound of formula (I) with poly-L-lysine.
Figure 8 shows the synthetic scheme for the glycoconjugate with poly-L-lysine (a=25) disclosed in Example 4.

### Detailed description of the invention

The object of the present invention is a carbohydrate derivative of formula (I): 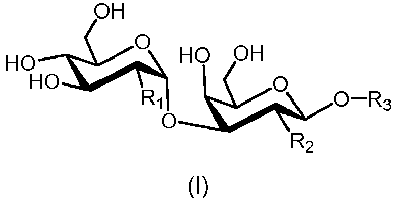 wherein:
R₁ is selected from OH and -NH-CO-R₅, wherein R₅ is C₁-C₃ alkyl and wherein one or more hydrogen atoms in R₅ may be replaced by a hydroxyl group;
R₂ is selected from OH and a group of formula (II): 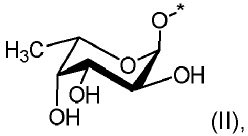 wherein * indicates the point of attachment of said group with the rest of the molecule;
R₃ is selected from R₄-Z, a group of formula (III) and a group of formula (IV): 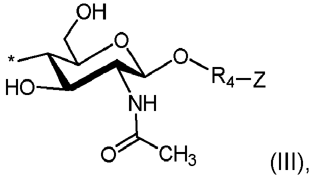 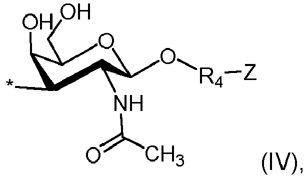 wherein
* indicates the point of attachment of said group with the rest of the molecule,
R₄ is a spacer group, and
Z is selected from -NH₂, -COOH, and -SH.

The authors of the present invention have developed new carbohydrate derivatives of formula (I), which, surprisingly, have high affinity towards natural anti-A/B antibodies despite the fact that they have a glucose or a glucosamine derivative at the nonreducing end, instead of a galactose or a N-acetylgalactosamine, as in natural A and B antigens.

### Definitions

The term "glycan", as is commonly used in the art, is a general term to designate carbohydrates, sugars, monosaccharides, oligosaccharides and polysaccharides. In particular, the term "glycan" is used herein to designate the carbohydrate portion of the carbohydrate derivative of formula (I) of the invention; in this regard, the terms "glycan" and "carbohydrate moiety" or "oligosaccharide moiety" are used interchangeably and are also used to designate the carbohydrate portion of the glycoconjugates.

The carbohydrate moiety in the compound of formula (I) is also occasionally referred to herein as "epitope", or "glycoepitope", or "glycotope" to denote that it is the active site which is recognized and bound to by the anti-A and/or anti-B antibodies. These carbohydrate moieties are also referred to as "mimetics", or "glycomimetics" to denote that they have different structure relative to the natural A/B antigens but are also recognized by the natural A/B antibodies.

The carbohydrate derivative of formula (I) is also referred to herein as "functionalized glycan", or "ligand" and is also generally represented as Glyc-sp-Z, wherein Glyc means the glycan or carbohydrate moiety, sp represents the R₄ spacer group, and Z is the functional group as defined above; i.e., the glycan "Glyc" in Glyc-sp-Z jointly represents the following glycans: 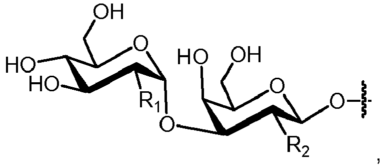 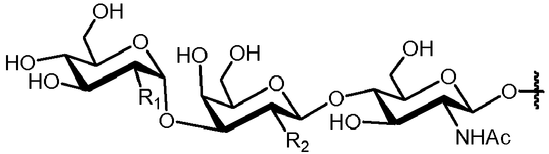 , and 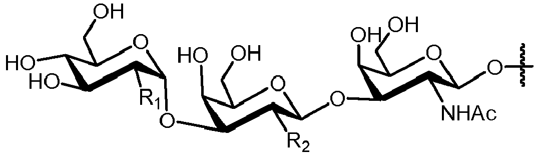 wherein R₁ and R₂ are as defined above, as well as the specific examples of each group.

In a particular embodiment, when -Z is -NH₂, the carbohydrate derivative of formula (I) may be represented as Glyc-sp-NH₂. In another particular embodiment, when -Z is -SH, the carbohydrate derivative of formula (I) may be represented as Glyc-sp-SH.

The term "glycoconjugate", as is conventionally understood, relates to carbohydrates covalently linked to other chemical species, in particular, to a polymeric substance, which is also referred to as polymeric backbone or support, for example, a protein, a peptide, a lipid, or a polysaccharide, among others. In particular, the glycoconjugates of the present invention comprise the functionalized glycan of formula (I) attached to a polymeric backbone. The glycoconjugate of the present invention is also interchangeably referred to herein as glycopolymer.

Cₙ-Cₘ alkyl means a linear or branched alkyl group of n to m carbon atoms. In particular, C₁-C₃ alkyl in the definition of the compound of formula (I) means an alkyl group of 1 to 3 carbon atoms, and includes therefore methyl, ethyl, n-propyl and isopropyl groups.

A linear alkylene group means a -(CH₂)ₙ- group, with variable values of n. For example, for n from 2 to 20, the alkylene group may be represented as C₂-C₂₀ alkylene, or for n from 2 to 10, the alkylene group may be represented as C₂-C₁₀ alkylene.

The term "alkylamino" as used herein means monoalkylamino group (-NHalkyl) or dialkylamino group (-N(alkyl)₂), i.e., it denotes an amino group substituted with one or two alkyl groups.

The term "alkoxy" as used herein means a -O-alkyl group.

The term "alkylthio" as used herein means a -S-alkyl group.

The term "hydroxyalkyl" as used herein means an alkyl group which is substituted with one or more hydroxyl groups.

The unspecified "alkyl" as used herein preferably relates to linear or branched C₁-C₆ alkyl, and more preferably to C₁-C₃ alkyl.

In general, the terms as used in this application, unless otherwise stated, shall be understood in their ordinary meaning as known in the art.

Along the present description, as well as in the claims, singular expressions, generally preceded by the articles "a", "an" or "the", are intended to include the plural forms as well, unless the context clearly indicates otherwise.

The terms "about" or "approximately" referred to amounts, as used herein, are meant to include the exact amount and also a certain deviation around the stated amount, namely of ±5%.

The numerical ranges disclosed herein are meant to include any number falling within the ranges and also the lower and upper limits.

### Compound of formula (I)

The object of the present invention is a carbohydrate derivative of formula (I): 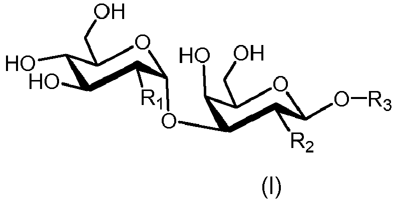 wherein R₁, R₂ and R₃ are as defined above.

The compound of formula (I) can also be abbreviatedly represented as Glc(R₁)α1-3(R₂)Galβ-R₃, where it is shown that the carbohydrate residue at the non-reducing end in the compound of formula (I) is a α-glucose (Glc), which is bound though a a (1→3) glycosidic linkage to a beta-galactose (Gal) unit.

In the compound of formula (I), as defined above, R₁ is either:
- OH, and then the nonreducing terminal carbohydrate is an α-glucose residue and the carbohydrate derivative of formula (I) is a mimetic of blood group B antigen; or
- -NH-CO-R₅, wherein R₅ is C₁-C₃ alkyl and wherein one or more hydrogen atoms in R₅ may be replaced by a hydroxyl group, and then the nonreducing terminal carbohydrate is an α-*N*-acylglucosamine residue and the carbohydrate derivative of formula (I) is a mimetic of blood group A antigen.

The R₂ group in the β-Gal residue may be either a OH or a fucose residue (of formula (II)).

The Glc(R₁)α1-3(R₂)Galβ- group is either directly bound to a linker of formula -R₄-Z, or is attached to another carbohydrate residue, which is itself bound to the linker of formula -R₄-Z. This optional additional carbohydrate is selected from a β-N-acetylglucosamine, bound at the 4 position (formula III), or a β-N-acetylgalactosamine, bound at the 3 position (formula IV).

In one embodiment, in the compound of formula (I), the group R₁ is selected from OH, -NH-CO-CH₃(*N*-acetyl or N-Ac), -NH-CO-CH₂CH₃ (*N*-propionyl) and -NH-CO-CH₂OH (N-glycolyl).

In one embodiment, in the compound of formula (I), the group R₂ is OH.

In another embodiment, in the compound or formula (I), the group R₂ is a fucosyl residue (group of formula (II)).

The linker group of formula -R₄-Z, which is attached at the reducing end of the carbohydrate of formula (I) contains a functional group -Z, which is either a -NH₂, or -COOH, or -SH group, which allows to covalently bind the active carbohydrate moiety to a suitable support, said functional group being linked to the carbohydrate though a spacer group R₄.

In one embodiment, Z is -NH₂.

In another embodiment, Z is -SH.

In another embodiment, Z is -COOH.

### Spacer

The spacer (R₄) is an arm connecting the oxygen atom at the reducing end of the carbohydrate moiety with the functional group Z. This linking arm maintains the active carbohydrate epitope spatially separated at a suitable distance from the polymeric support in the glycoconjugate of the compound of formula (I). In this regard, therefore, the selection of a particular spacer is not critical insofar as it is suitable for this function.

Typically, the spacer R₄ is a linear chain, for example, of 2 to 20 atoms, which is not immunogenic and also not reactive, typically, under the working conditions of the compound (I), for example, for forming the glycoconjugate with a polymeric support.

The spacer R₄ is typically a linear alkylene, for example, a C₂-C₂₀ linear alkylene, preferably a C₂-C₁₀ linear alkylene, which is unsubstituted or substituted, for example, mono-, di- or trisubstituted, for example, by groups such as alkyl, alkylamino, hydroxyl, alkoxy, hydroxyalkyl, halogen, thiol, alkylthio, or cyano, among others; and wherein some non-consecutive -CH₂- groups in the alkylene chain, for example, up to 5 non-consecutive -CH₂- groups, preferably up to 3 non-consecutive -CH₂- groups may be replaced by other groups, by interrupting the alkylene chain with, for example, ether, thioether, ketone, amine, and/or an amide groups, among others.

In one embodiment, the spacer R₄ in the compound of formula (I) is a linear C₂-C₁₀ alkylene group, preferably unsubstituted, and wherein up to 3 non-consecutive -CH₂- groups may be replaced by a group selected from -O-, -S-, - N(H)-, -N(R₅)-, -N(OR₅)-, -N(H)-CO-, -CO-N(H)-, -N(R₅)-CO- and -CO-N(R₅)-, wherein R₅ is a C₁-C₃ alkyl group.

In one particular embodiment, the spacer R₄ is a group of formula - (CH₂)₂₋₈-NH₂, for example, a group of formula -(CH₂)₃-NH₂, which is the one used in the preparative examples, but other spacers, as defined above, can be equally suitable.

In one embodiment, the object of the present invention is a carbohydrate derivative of formula (I): 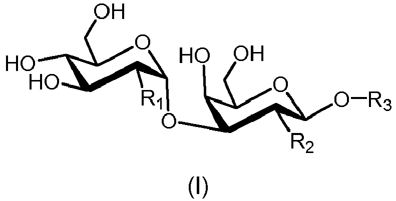 wherein:
- R₁ is selected from OH and -NH-CO-R₅, wherein R₅ is C₁-C₃ alkyl and wherein one or more hydrogen atoms in R₅ may be replaced by a hydroxyl group; preferably R₁ is selected from the group consisting of OH, -NH-CO-CH₃, -NH-CO-CH₂CH₃ and -NH-CO-CH₂-OH.
- R₂ is selected from OH and a group of formula (II): 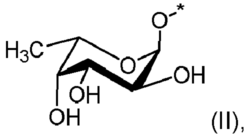 wherein * indicates the point of attachment of said group with the rest of the molecule;
   and preferably R₂ is a group of formula (II);
- R₃ is selected from R₄-Z, a group of formula (III) and a group of formula (IV): 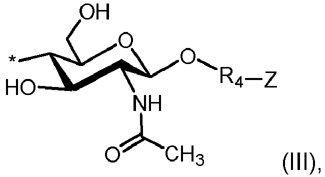 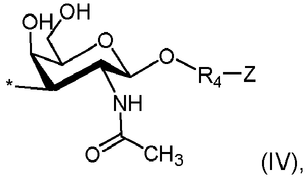 wherein
   * indicates the point of attachment of said group with the rest of the molecule, R₄ is a spacer group;
   preferably R₄ is a C₂-C₂₀ linear alkylene, more preferably a C₂-C₁₀ linear alkylene, which is unsubstituted or substituted, for example, mono-, di- or trisubstituted, by groups independently selected from alkyl, alkylamino, hydroxyl, alkoxy, hydroxyalkyl, halogen, thiol, alkylthio and cyano, and wherein up to 5 non-consecutive -CH₂- groups in the alkylene, preferably up to 3 non-consecutive -CH₂- groups, may be replaced by other groups, interrupting the alkylene chain with ether, thioether, ketone, amine, and/or an amide groups; more preferably R₄ is a linear C₂-C₁₀ alkylene group, preferably unsubstituted, wherein up to 3 non-consecutive -CH₂- groups may be replaced by a group selected from -O-, -S-, -N(H)-, -N(R₅)-, -N(ORs)-, -N(H)-CO-, -CO-N(H)-, - N(R₅)-CO- and -CO-N(R₅)-, wherein R₅ is a C₁-C₃ alkyl group;
   still more preferably, the spacer R₄ is a group of formula -(CH₂)₂₋₈-NH₂, and still more preferably R₄ is a group of formula -(CH₂)₃-NH₂; and Z is selected from -NH₂, -COOH and -SH, preferably is -NH₂

In one particular embodiment of the invention, when R₂ is OH, then R₃ is R₄-Z.

Preferred examples of the compound of formula (I) are the following:

**TABLE 1**

| **Compound:** | **Structure** | **Mimetic of:** |
|---|---|---|
| 1 | Glcα1-3(Fucα1-2)Galβ1-4GlcNAcβ-R | Group B antigen |
| 2 | Glcα1-3(Fucα1-2)Galβ-R | Group B antigen |
| 3 | Glcα1-3Galβ-R | Group B antigen |
| 4 | Glcα1-3(Fucα1-2)Galβ1-3GalNAcβ-R | Group B antigen |
| 5 | GlcNAcα1-3(Fucα1-2)Galβ1-4GlcNAcβ-R | Group A antigen |
| 6 | GlcNPropionylα1-3(Fucα1-2)Galβ1-4GlcNAcβ-R | Group A antigen |
| 7 | GlcNGlycolylα1-3(Fucα1-2)Galβ1-4GlcNAcβ-R | Group A antigen |
| 8 | GlcNAcα1-3(Fucα1-2)Galβ-R | Group A antigen |
| 9 | GlcNPropionylα1-3(Fucα1-2)Galβ-R | Group A antigen |
| 10 | GlcNGlycolylα1-3(Fucα1-2)Galβ-R | Group A antigen |
| 11 | GlcNAcα1-3Galβ-R | Group A antigen |
| 12 | GlcNPropionylα1-3Galβ-R | Group A antigen |
| 13 | GlcNGlycolylα1-3Galβ-R | Group A antigen |
| 14 | GlcNAcα1-3(Fucα1-2)Galβ1-3GalNAcβ-R | Group A antigen |
| 15 | GlcNPropionylα1-3(Fucα1-2)Galβ1-3GalNAcβ-R | Group A antigen |
| 16 | GlcNGlycolylα1-3(Fucα1-2)Galβ1-3GalNAcβ-R | Group A antigen |
| | (R= -O-R₄-Z) | |

The compounds where R₁ is a *N*-acyl group are mimetics of blood group A antigen, while those where R₁ is OH are mimetics of blood group B antigen.

### Process for preparing of the compound of formula (I):

The compound of formula (I) can be prepared using conventional synthetic methods, well-known to the skilled in carbohydrate synthesis.

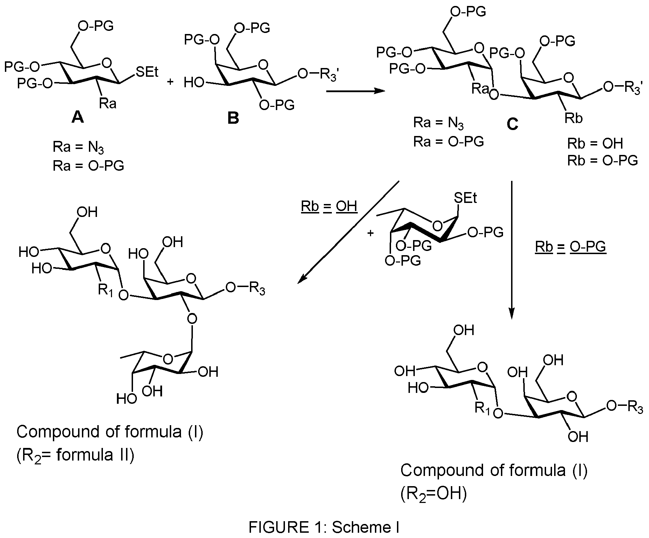

A possible general synthetic route for preparing the compounds of formula (I) is depicted in Scheme I (Figure 1).

In Scheme I, PG are conventional protecting groups, for example, each one is typically independently selected from acetyl (Ac, CH₃-CO-), benzyl (Ph-CH₂-), or benzylidene (Ph-CH=); and R₃' is selected from R₄-Z', a group of formula (III) and a group of formula (IV), wherein the group of formula (III) and the group of formula (IV) are as defined above, but wherein the OH groups in the carbohydrate moiety in formulas (III) and (IV) are protected, for example, with the same protecting groups as defined above, and wherein Z' is a group of formula Z as defined above which is protected.

Benzylidene protecting group, as is well known in the art, can be used for simultaneously protecting two spatially proximate OH groups, for example, at C-4 and C-6 of a galactose moiety:

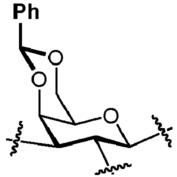

The Z group, as above defined, is -NH₂, -SH or -COOH. Any customary protecting group for these functional groups, as are well known in the art, may be used. A suitable protecting group for -NH₂ and -SH is, for example, an acyl group, such as acetyl, or trifluoroacetyl, among others. A suitable protecting group for -COOH is an ester, for example, methyl, *t*-butyl or benzyl ester, among others.

In Scheme I, when, in the starting material of formula **A**, Rₐ is an azide group (N₃), the compounds of formula (I) wherein R₁ is an acyl group (of formula -NH-CO-R₅, as defined above) can be obtained. Thus, the azide group is typically reduced to an amine group and then modified with the suitable acyl residue. When, in the starting material of formula **A**, Rₐ is a protected hydroxyl (such as, for example, with the benzyl group, i.e., OBn), the compounds of formula (I) wherein R₁ is OH can be obtained.

In Scheme I, the intermediate compounds of formula **C** wherein the OH group at the position C-2 of the galactose residue is protected (R_{b}=O-PG) lead to the compounds of formula (I) wherein R₂=OH. When this position is left unprotected (R_{b}=OH), it allows for linking a fucose residue through a a (1→2) glycosidic linkage at this position, to provide the compounds of formula I wherein R₂ is a fucose residue (of formula II, as defined above).

Therefore, it is understood that, depending on the different alternatives, according to the different values of the moieties R₁, R₂ and R₃, the skilled in the art will have no difficulty in suitably adapting the above-described synthetic route to the preparation of a specific compound of formula (I).

In Scheme I, it is to be understood that the compound of Formula (I) directly obtained after the carbohydrate synthesis may have a spacer and linking group attached at the reducing end which is different from the one defined above, and is subsequently converted, using conventional organic synthetic methods, to the linking group -R₄-Z, as defined above.

Another aspect of the invention is, therefore, a process for the preparation of a compound of formula (I), characterized in that it comprises the following steps:
a) Preparing a compound of formula **C** from a compound of formula **A** and a compound of formula **B**: 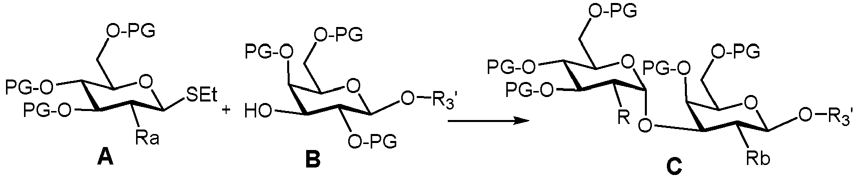 wherein
   PG are protecting groups;
   Rₐ is azide or O-PG;
   R₃' is selected from R₄-Z', a group of formula (III) and a group of formula (IV), wherein the group of formula (III) and the group of formula (IV) are as defined above, but wherein the OH groups in the carbohydrate moiety in formulas (III) and (IV) are protected, and wherein Z' is a group of formula Z as defined above which is protected;
   R_{b} is OH or O-PG; and
b)
   b1) when R_{b}=OH, linking a protected fucose residue through a a (1→2) glycosidic linkage at this position to provide, after deprotection, the compound of formula (I) wherein R₂ is a group of formula II;
   b2) when R_{b}=O-PG, deprotecting the compound of formula **C** to provide a compound of formula (I) wherein R₂ is OH;
wherein the definitions of protecting groups and different conditions are as discussed above.

In particular, the preparation of the compounds of formula (I) comprising the fucose residue can be performed, for example, analogously as disclosed in Kunetskiy et al., Synthesis of blood group A and B (type 2) tetrasaccharides. A strategy with fucosylation at the last stage, Carbohydrate Research, 2020, Vol. 498, 108192, where the preparation of analogous derivatives of the natural blood group A and B antigens is described, i.e., comprising a α-Gal at the non-reducing end.

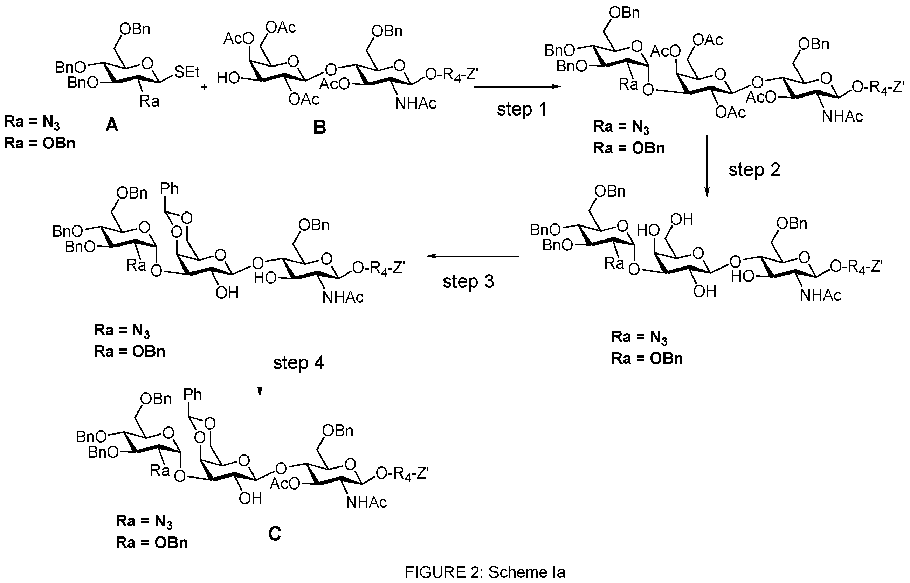

Thus, for example, for preparing the compounds of formula (I) wherein R₂ is a group of formula (II), and R₃ is a group of formula (III), the route depicted in Scheme Ia (Figure 2) can be followed.

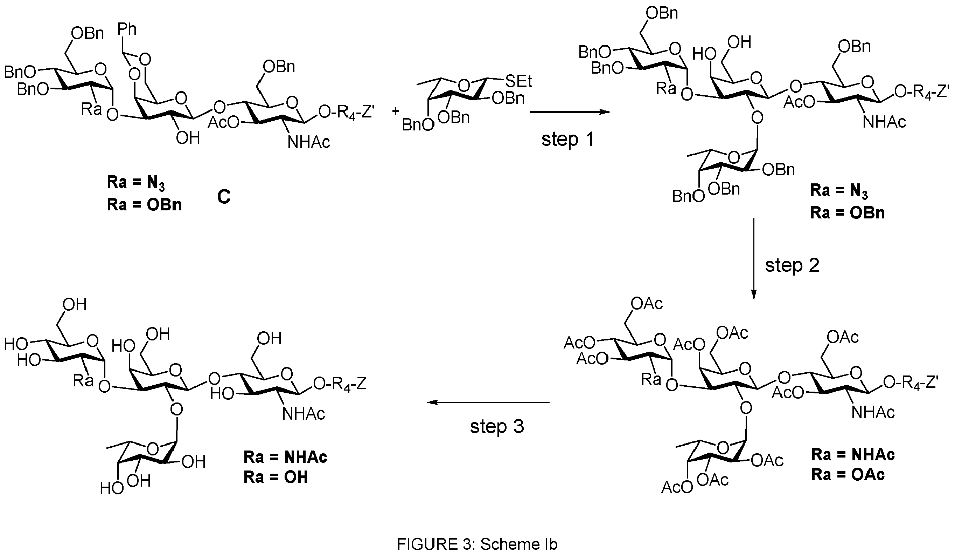

The compound of C is then coupled with the fucose residue at the C-2 position of the galactose residue, to provide, after deprotection, the compound of formula (I), according to the route depicted in Scheme Ib (Figure 3)

Compounds 1, 5, 6 and 7 of Table 1, for example, can be prepared according to this route.

Analogously, for preparing the compounds of formula (I) wherein R₂ is a group of formula (II), and R₃ is a group of formula (IV), an analogous procedure as depicted in Schemes Ia and Ib can be followed, but using, for example, the following compound of formula B as starting material:

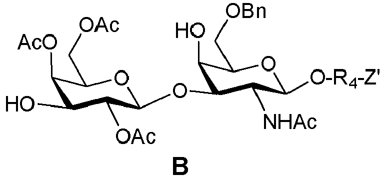

In this way, for example, compounds 5, 14, 15 and 16 of Table 1 can be obtained.

Also analogously, for preparing the compounds of formula (I) wherein R₂ is a group of formula (II), and R₃ is a group of formula R₄-Z, an analogous procedure as depicted in Schemes Ia and Ib can be followed, but using, for example, the following compound of formula B as starting material:

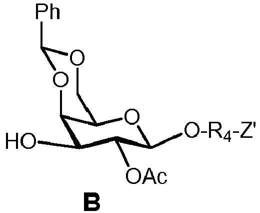

In this way, for example, compounds 3, 9, 10 and 11 of Table 1 can be obtained.

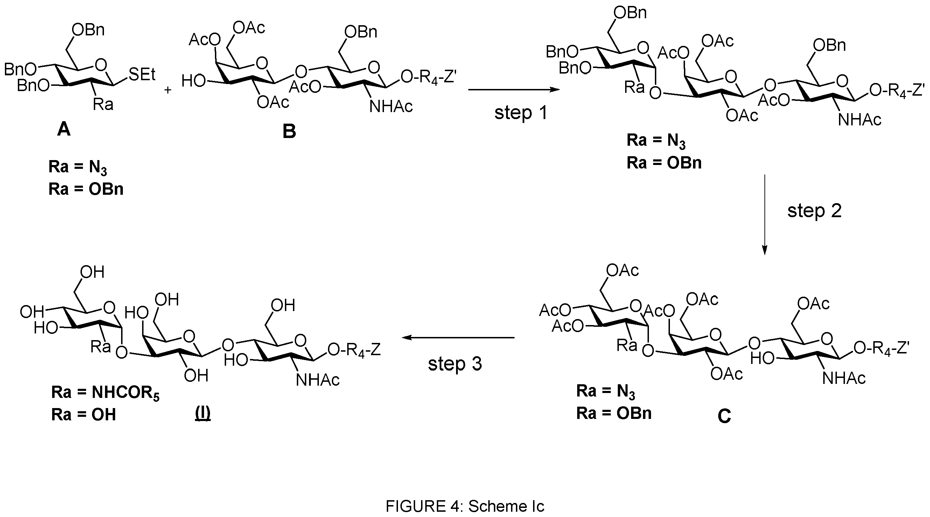

On the other hand, the compounds of formula (I) wherein R₂ is OH and R₃ is a group of formula (III), can be prepared by the route depicted in Scheme Ic (Figure 4).

Analogously, for preparing the compounds of formula (I) wherein R₂ is OH, and R₃ is a group of formula (IV), an analogous procedure as depicted in Scheme Ic can be followed, but using, for example, the following compound of formula **B** as starting material:

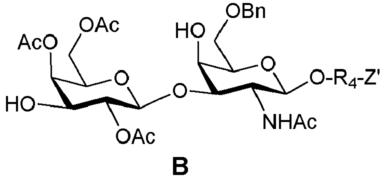

Also analogously, for preparing the compounds of formula (I) wherein R₂ is OH, and R₃ is a group of formula R₄-Z, an analogous procedure as depicted in Scheme Ic can be followed, but using, for example, the following compound of formula **B** as starting material:

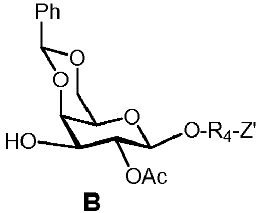

In this way, for example, compounds 3, 11, 12 and 13 of Table 1 may be prepared.

In the Scheme I, the compounds of formula (I) comprising a fucose (i.e., when R₂ is a group of formula II) are prepared by adding the fucose moiety in the final step, to the previously prepared intermediate of formula C. It is to be understood that other alternative synthetic routes may be equally suitable, for example, by first obtaining a fucosylated intermediate B derivative and, subsequently, coupling it to the protected alpha-glucose starting material of formula A. It is well within the capabilities of the skilled in carbohydrate synthesis to identify other alternative routes to synthesise the compound of formula (I).

### Glycoconjugates of compounds of formula (I)

Another aspect of the invention is a glycoconjugate of the compound of formula (I). The glycoconjugate of the invention comprises the compound of formula (I), more particularly, it comprises the compound of formula (I) and a polymeric backbone or support. In the glycoconjugate, the compound of formula (I) is covalently bound to a polymeric backbone or support, more specifically, the compound of formula (I) is bound through the -Z functional group to the polymer.

It is understood that the -NH₂, -SH and -COOH functional groups in the compound of formula (I) are typically converted to -NH-, -S-, and -CO- or -COO- groups, respectively, in the glycoconjugate, when the covalent bond is formed between said functionalized glycan and the polymer. Therefore, when it is defined that the glycoconjugate "comprises" the compound of formula (I), it is to be understood that the -Z group has been modified in this way.

As is well known in the art, such covalent binding can be performed directly between the ligand and the polymer or they can be linked through a suitable additional linking agent (or crosslinker) or by including a reactive group on either the polymer or the ligand or both. Said reactive groups have therefore the purpose of activating either the polymer or the compound of formula (I) in order to allow or facilitate the reaction between both moieties.

It is to be understood, therefore, that the final glycoconjugate may comprise an additional linking fragment between both parts, as is illustrated below.

The glycoconjugate of the present invention is prepared by linking the carbohydrate derivative of formula (I) to a suitable polymeric backbone through a covalent bond. More particularly, said glycoconjugate is formed with a multitude of compounds of formula (I) linked through a covalent bond to a multitude of functional groups on the polymer backbone.

Suitable polymers to be used as backbone or support for the glycoconjugate of the invention are, for example, homologous and heterologous α-amino acid polymers (homo and heteropolypeptides), proteins, lipids, nucleic acids, acrylic acid polymers, methacrylic acid polymers, acrylic/methacrylic acid copolymers, polysaccharides such as chitosan or agarose, polyethylene, polypropylene, polystyrene, polyvinyl butyrate, poly (vinyl chloride), or dendrimers, for example, among others. Suitable homologous α-amino acid polymers are, for example, polylysine, polyarginine, polyornithine, polyglutamine, polyasparagine, polyglutamic acid or polyaspartic acid. Said poly α-amino acids may be linear or branched.

The polymer may be used as such or may be combined with an additional material, such as glass, ceramics, or metals, for example. In one embodiment, the polymer is included in magnetic nanoparticles (MNPs), which, as is well known in the art, are characterized in that they comprise a magnetic material in the core, such as iron, nickel or cobalt, and a polymeric shell, for example, of polyacrylic acid or other polymers, which bears suitable functional groups to be bound to the ligand.

The selection of the particular polymer or the particular form of it for preparing the glycoconjugate of the invention will be based on the desired end use of the glycoconjugate.

In one embodiment, the polymer in the glycoconjugate of the invention is selected from polyacrylic acid, a polysaccharide resin and an α-aminoacid homopolymer, preferably the polymer is selected from polyacrylic acid, agarose and polylysine.

The polylysine is either linear or branched poly-L-lysine, preferably linear poly-L-lysine.

In one embodiment, the polymer in the glycoconjugate is included in a magnetic nanoparticle.

For a glycoconjugate prepared using a given polymer, it is possible to bind a single compound of formula (I) to the multiple reactive sites on the polymer or, alternatively, different compounds of formula (I) may be bound to the polymer to obtain a polyfunctional glycopolymer. Preferably, a single compound of formula (I) is comprised in a given glycopolymer.

Furthermore, for a particular glycoconjugate, comprising a given polymer and a given compound of formula (I), different specific products can be obtained depending on the polymerization degree of the polymer, i.e., its molecular weight.

As is well known in the art, said polymers suitable to prepare glycoconjugates are characterized in that they comprise functional groups in their repetitive polymeric structure, such as hydroxyl, carboxyl or amino groups, among others, through which a covalent bond is formed with the functional group on carbohydrate ligand of the invention, in particular, with the amine, the carboxyl, or the thiol functional group on the ligand. Usually, the polymer needs to be activated with a compound that is reactive towards said functional group on the ligand. The activated complex can then generate a covalent linkage with said compound, resulting in immobilization of the carbohydrate epitope on the polymer, to form the glycoconjugate.

There are different suitable methods for activating the polymeric support, which vary depending on the specific polymer, as is well known in the art. Different activation methods, for example, are described in the chapter Immobilization of Ligands on Chromatography Supports, in: Bioconjugate Techniques, G.T. Hermanson, Ed., Chapter 15, p: 589-740.

For example, some well-known activation strategies for linking the polymer with an amine group on the glycan are, for example, activation of carboxylate groups on the polymer as *N*-hydroxysuccinimide (NHS) ester groups, which afford amide bonds with the ligand; aldehyde activated polymers which afford secondary amine bonds with the ligand; azlactone activated polymers, which afford amide bonds with the ligand; activation of hydroxyl groups with 1,1'-carbonyl diimidazole (CDI) to form reactive imidazole carbamates, which afford carbamate bonds with the ligand; or activation of carboxylate groups on the polymer as 4-nitrophenyl ester groups, to afford amide bonds with the ligand, among many other options.

Another well-known strategy for linking the ligand of formula (I) to the polymer support is by using a linker, which is a bifunctional moiety, either homobifunctional or heterobifunctional that makes possible to link both moieties. For example, when both the support and the ligand bear an amine functional group, a common homobifunctional linker is a succinimidyl diester, for example, adipic acid succinimidyl diester or disuccinimidyl suberate, among others, which can afford two amide bonds at the two ends of the molecule, to form the glycoconjugate. In this case, either the ligand can be first activated and then reacted with the polymer, or the polymer can be first activated and then reacted with the ligand.

Another well-known option for linking a glycan bearing a thiol functional group with a polymeric support bearing an amine group is by first activating the amine groups on the support by chloroacetylation, and subsequent reaction between the chloroacetylated polymer and the thiol functional groups on the ligand to produce the glycoconjugate. This strategy is disclosed, for example, in Duthaler et al., Chimia, 2010, 64(1-2), 23-28. Analogously, -OH functional groups on the polymer may also be activated by chloroacetylation, to be linked to a thiol-functionalized glycan.

Other suitable activating methods for preparing glycoconjugates are also disclosed, for example, in the international patent application WO-A-2018/167320.

The different strategies for the preparation of glycopolymers are well known in the art and it is well within the capabilities of the skilled in the art to choose the most suitable method and conditions for each case.

The load degree of the ligand on the polymer means the percentage of the functional groups on the polymer, which are attached to the active saccharide of the invention. In general, loads of from 2% to 90% are used, preferably from 5% to 50%, and more preferably from 10% to 40%. The remaining not bound functional groups on the polymer are usually capped, i.e., they are reacted with a suitable capping substance, with the purpose of suppressing the reactivity of the remaining free functional groups on the polymer. The capping agent to be used depends on the particular functional group on the polymer. The selection of the specific capping agent is not critical, as long as it can be easily bound with high yield to the free reactive sites on the polymer and it produces stable, non-reactive capped ends. The capping molecule is preferably hydrophilic to avoid loss of solubility in water, not antigenic, and not highly charged. It is therefore well within the capabilities of the skilled in the art to choose a suitable capping agent in each case. Commonly used capping agents are, for example, glycolic acid, gluconic acid, succinic acid, ethanol amine or other amine derivatives, thio-compounds such as thioglycerol, among many others.

In one embodiment, the glycoconjugate of the invention comprises the compound of formula (I) linked to a polyacrylic acid backbone. The glycoconjugate thus formed is a polyacrylamide.

In one particular embodiment, the glycoconjugate has the formula (V) as shown below: 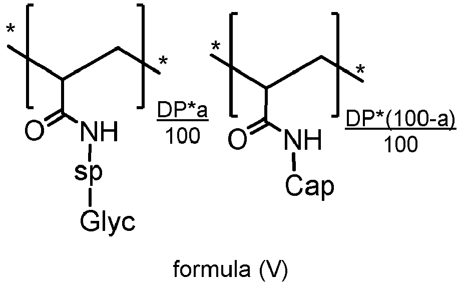 wherein "Cap" means a capping agent, "DP" represents the degree of polymerization of the polymer, expressed as number of monomers, and "a" represents the load percentage of the carbohydrate in the glycoconjugate. The DP is preferably comprised between 50 and 1100, and more preferably between 100-1000, and the load percentage is preferably comprised between 5 and 80, more preferably between 10 and 70.

Any suitable capping agent, as known in the art, may be used. For example, a suitable capping agent is ethanol amine, and the polyacrylamide glycoconjugate has the following formula:

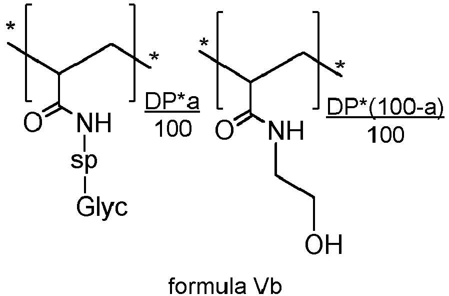

In one preferred embodiment, the glycoconjugate of formula (V) and (Vb) is prepared from a compound of formula (I) which is selected from compounds 1 to 16 of Table 1.

For preparing the glycoconjugate with a polyacrylic acid backbone, the acidic -COOH groups on the polyacrylic acid can be activated, for example, with N-hydroxysuccinimide (NHS), forming *N*-succinimide ester groups, or can be activated as 4-nitrophenyl ester groups, for example, as disclosed in Tuzikov et al., 40 years of glyco-polyacrylamide in glycobiology, Glycoconj. J., 2021, 38, 89-100.

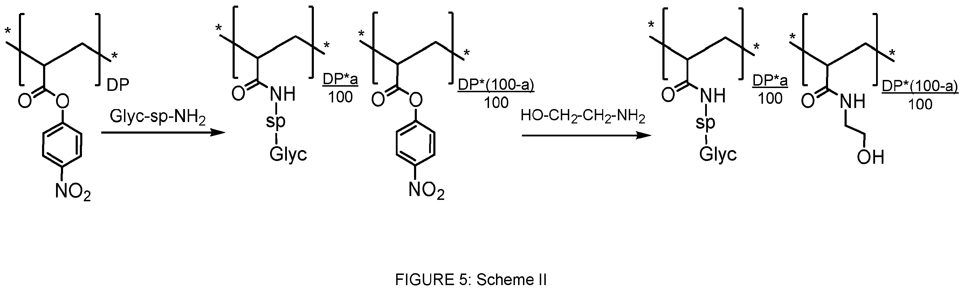

The glycoconjugate with polyacrylic acid may be prepared, for example, as depicted in Scheme II (Figure 5), where the -COOH groups of the polyacrylic acid are activated as 4-nitrophenyl ester groups and the capping agent is ethanolamine.

Other activation methods of polyacrylic acid may also be suitable for preparing the glycoconjugate with the functionalized glycan of the invention.

In another embodiment, the glycoconjugate comprises the compound of formula (I) linked to agarose as polymeric backbone. Agarose, as is well known, is a linear polysaccharide made up of the repeating unit of agarobiose and is commercially available as resin beads. Crosslinked forms of agarose are commercially available under the denomination Sepharose^{®}. For preparing the glycoconjugates, typically, activated forms of agarose are used, which are commercially available. These activated forms are available as resin beads of different sizes.

Suitable activated agarose forms are, for example, *N-*hydroxysuccinimide (NHS)-activated agarose, aldehyde-activated agarose, azlactone-activated, or 1,1'-carbonyl diimidazole (CDI) activated agarose, among others. For example, the linking between the ligand and the NHS-activated agarose may be performed as represented in the following scheme:

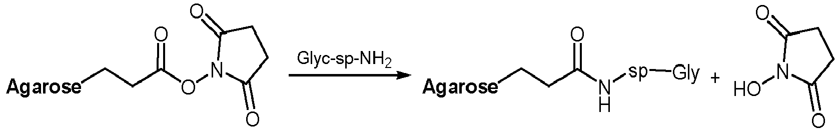

Preferred forms of agarose are those having molecular weight comprised between 25-1000 KDa.

In one preferred embodiment, the glycoconjugate with agarose is prepared from a compound of formula (I) is selected from the compounds 1 to 16 of Table 1.

In another embodiment, the glycoconjugate of the invention comprises the compound of formula (I) linked to a polylysine backbone, in particular to poly-*L*-lysine.

In one particular embodiment, the glycoconjugate with poly-*L*-lysine has the following formula: 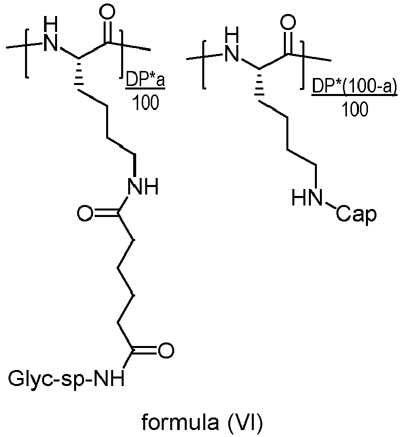 wherein "Cap" means a capping agent, "DP" represents the degree of polymerization of the polymer, expressed as number of lysine units, and "a" represents the load percentage of the carbohydrate in the glycoconjugate.

The polylysine may be linear or branched, and is preferably linear.

Suitable degrees of polymerization of polylysine are comprised between about 50 and 2000, preferably comprised between 100 and 1000. Preferred polylysine of degrees of polymerisation are about 100, about 600 and about 1000, which are commercially available, in the form of the hydrobromide salt. The poly-*L*-lysine hydrobromide may be synthetized, for example, via ring-opening polymerization of *N*-carboxy-anhydrides using N6-trifluoroacetyl-*L*-lysine as precursor, as disclosed in Hadjichristidis et al., Chem. Rev., 2009, 11, 5528-5578.

The ligand loading on the polylysine is typically comprised between 2% and 90%, preferably comprised between 5% and 50%, and more preferably comprised between 10% and 40%.

Any suitable capping agent, as known in the art, may be used for the glycoconjugate with polylysine. For example, a suitable capping agent is glycolic acid, forming glycolic amide terminal groups, and the glycoconjugate has the following formula:

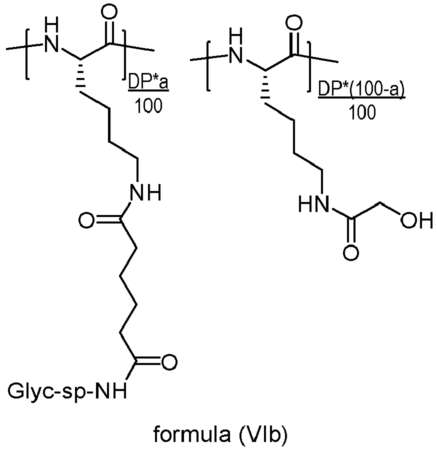

In one preferred embodiment, the glycoconjugate of formula (VI) and (Vlb) is prepared from a compound of formula (I) is selected from the compounds 1 to 16 of Table 1.

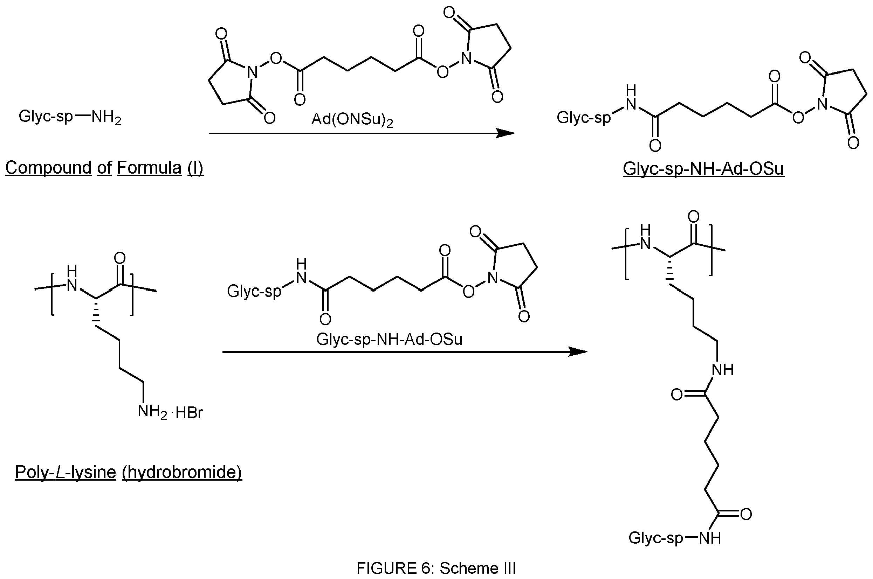

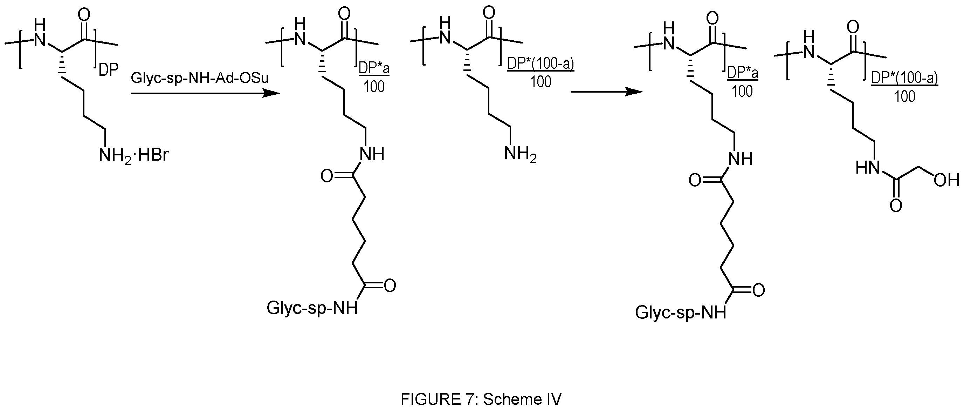

For preparing the glycoconjugates of formula (VI) and (Vlb), the ligand of formula Glyc-sp-NH₂ is bound to the amine groups on the side chain of polylysine using a *N*-hydroxysuccinimidyl diester linker, in particular, with adipic acid diester. Preferably, the ligand is first activated by reaction with the diester, and then, the activated compound is reacted with the polylysine (as hydrobromide), as shown in Scheme III (Figure 6). The preparation of the glycoconjugate is represented in Scheme IV (Figure 7).

The capping with glycolic acid may be performed, for example, in two steps, by first reacting the free amino groups on the glycoconjugate with an activated and protected glycolic acid (glycolic acid acetate *N*-hydroxysuccinimide ester) and then deprotecting the acetate protected glycolic amide groups, to obtain the final capped product.

In another embodiment, the glycoconjugate of the invention comprises the compound of formula (I) linked to a magnetic nanoparticle. Magnetic nanoparticles (MNPs) are well known in the art, and they are characterized in that they comprise a magnetic material in the core, such as iron, nickel or cobalt, and typically a polymeric shell, which bears suitable functional groups to be bound to the ligand, using common linking methods as described above. For example, N-hydroxysuccinimide activated magnetic nanoparticles may be linked to the functionalized glycan of the invention, according to the following reaction:

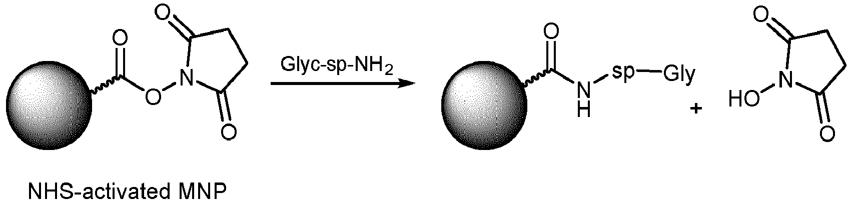

NHS-activated magnetic nanoparticles are commercially available, for example, from the company CD Bioparticles. Other type of activated magnetic nanoparticles and other linking mechanisms with the glycomimetic of the invention may be also suitable, and adapting the process to the specific activated magnetic nanoparticle is well within the capabilities of the skilled in the art.

Furthermore, said glycoconjugates or glycopolymers also are meant to include any functionalized surface, bearing the active mimetics of formula (I), which allow for identification of the antibodies from blood samples.

### Uses of the glycoconjugate

The glycoconjugates of the invention, comprising the glycan of formula (I), surprisingly, have high affinity towards natural anti-A/B antibodies, despite the fact that they carry active epitopes which differ from the natural A/B antigens, in particular, despite the fact that they comprise a glucose or a glucosamine derivative at the nonreducing end, instead of a galactose or a *N-*acetylgalactosamine, as in natural A and B antigens, contrary to what was disclosed so far in the art.

Furthermore, not only the new epitopes are effective for binding natural anti-A/B antibodies, but they are even more active than the natural antigens.

This is shown in the assays disclosed in Examples 5 to 8, where their efficacy for inhibition and removal of anti-A/B antibodies was tested, both in normal human serum from blood group A or B and in pure fractions of human anti-A/B antibodies, as well as their use for purification of human polyclonal anti-A/B antibodies.

This outstanding affinity towards anti-A/B antibodies may be used for inhibiting and/or removing anti-A and/or anti-B antibodies from samples of blood and blood derivatives. This is useful for many applications, for example, to allow transfusion and organ transplants between incompatible blood donors including living-donor organ transplantation or for inhibition and elimination of anti-A and anti-B antibodies during the process of purification and fractionation of blood products, among other applications.

The use of the glycoconjugates for avoiding rejection in organ transplantation is suitable, for example, for kidney, liver, skin, pancreas, cornea, bone marrow or heart transplants. To this end, the anti-A and/or anti-B antibodies can be inhibited/removed either *ex vivo* or *in vivo,* or complementarily *ex vivo* and *in vivo.* For *ex vivo* removal, the blood from an organ transplant recipient is withdrawn from the subject and then contacted with the glycoconjugate of the invention to remove the anti-A and/or anti-B antibodies, and then the treated blood is reinfused into the subject. In the *in vivo* alternative, the inhibition/removal of the anti-A and/or anti-B antibodies is performed intracorporeally, by administering a suitable glycoconjugate which is able to inhibit/remove the circulating antibodies in the subject.

For this intracorporeal use, the polymer in the glycoconjugate is typically a biodegradable, biocompatible polymer. Suitable polymers for this use are poly amino acids, for example, polylysine or polyarginine, among others. One particularly preferred glycoconjugate for intracorporeal use is poly-*L*-lysine glycoconjugate, as poly-*L*-lysine has proved to be safe and not immunogenic after *in vivo* administration (Katopodis et al., J. Clin. Invest., 2002, 110, 1869-1877).

On the other hand, the applications related to the purification and fractionation of blood and blood derivatives samples, or the identification of the antibodies from blood or blood derivatives samples are performed *in vitro* or *ex vivo.*

A suitable glycoconjugate may be chosen for each particular purpose, comprising a suitable glycan, either mimetic of blood group A antigen or mimetic of blood B antigen, depending on the antibodies to be removed or detected.

Another aspect of the invention is, therefore, the use of the glycoconjugate of the invention for inhibiting and/or removing anti-A and/or anti-B antibodies from samples of blood or other blood derivatives and by-products. Such blood derivatives and by-products are, for example, serum, plasma, fractioned plasma, or immunoglobulin preparations, among others. According to this aspect, it is understood that the samples of blood and/or blood derivatives/by-products are treated *in vitro* or *ex vivo* with the glycoconjugate, and no treatment of living being is performed.

Typically, a method for removing the anti-A and/or anti-B antibodies from samples of blood or other blood derivatives and by-products comprises contacting the sample with the glycoconjugate during sufficient time to allow the antibodies to be bound by the active epitopes on the glycoconjugate, and then separating the glycoconjugate from the sample. The glycoconjugate is typically made of a solid polymeric support, which is, for example, in the form of solid microbeads for facilitating the contact with and subsequent separation from the treated sample.

A method for inhibiting the anti-A and/or anti-B antibodies from said samples is analogous, but the sample may not be separated from the inactivated antibodies, which remain in the sample bound to the glycoconjugate.

The high-affinity binding of the anti-A/B antibodies by the glycoconjugate of the invention can also be used for detecting those antibodies in test samples. For several *in vitro* applications, the glycomimetic of the invention may be attached to any suitable surface or device adapted to antibody detection.

Another aspect of the invention is the glycoconjugate of the invention for use in medicine, in particular, for use in the treatment and/or prevention of rejection or haemolytic reactions in subjects undergoing organ transplantation or blood transfusion, respectively, from incompatible donors.

Another aspect of the invention is a method for treating and/or preventing rejection or haemolytic reactions in a subject in need thereof undergoing organ transplantation or blood transfusion, respectively, from incompatible donors, comprising the step of administering a therapeutically effective amount of the glycoconjugate of the invention.

Another aspect of the invention is the use of the glycoconjugate of the invention for the preparation of a medicament for the treatment and/or prevention of rejection or haemolytic reactions in subjects undergoing organ transplantation or blood transfusion, respectively, from incompatible donors.

Incompatible donors are meant to be organ donors or blood donors.

According to this aspect, related to the treatment or prevention of rejection or haemolytic reactions in a human being, the glycoconjugate of the invention comprises a biodegradable and biocompatible polymer. Preferred polymers are homo poly α-amino acids, for example, selected from polylysine, polyarginine, polyornithine, polyglutamine, polyasparagine, polyglutamic acid and polyaspartic acid. Particularly preferred is polylysine, particularly poly-L-lysine, as defined above.

Typically, the glycoconjugate as therapeutic agent is administered by intravenous, subcutaneous, or intramuscular injection.

"Biodegradable" as used herein means metabolic biodegradability so that the polymer, after being administered, is biofragmented and removed from the organism. "Biocompatible" as used herein means that the polymer is safe and nontoxic and therefore suitable for being administered to living organisms.

The terms "inhibition" and "removal" may be used interchangeably herein. Inhibition refers to the fact that the antibodies, once bound to the active epitopes on the glycoconjugate of the invention, are inactivated and are not able to further bind to the natural anti-A/B antigens, even if they remain present in the sample. Removal refers to the fact the antibodies are eliminated from the treated sample, either after *in vitro* or *ex vivo* processes, where the treated sample is collected and the antibodies are retained on the solid glycopolymer, or after *in vivo* intracorporeal inhibition, when immunocomplexes are then metabolized and also removed from blood stream.

As used herein, the subjects undergoing organ transplantation or blood transfusion which are to be treated with the glycoconjugate of the invention are meant to be human beings.

Incompatible donors, as used herein, means subjects having ABH-incompatible antigens.

The glycoconjugate of the invention may be administered prior to the subject receiving an ABO-incompatible transplant or blood transfusion, or alternatively or complementarily, the treatment may be administered after a subject receives an ABO incompatible transplant or blood transfusion.

### Pharmaceutical compositions

Another aspect of the invention is a pharmaceutical composition comprising the glycoconjugate of the invention and at least one pharmaceutically acceptable excipient.

It is understood that the glycoconjugate suitable for preparing a pharmaceutical composition is as defined above for the medical treatment, i.e., the polymer of the glycoconjugate is typically biocompatible and biodegradable. Preferably, the polymer is a poly α amino acid, more preferably is a polylysine, still more preferably a poly-*L*-lysine and still more preferably is a poly-*L*-lysine as defined above under the Glycoconjugate section.

The pharmaceutical composition of the invention is prepared according common procedures, well known in the art, and using common and widely available excipients, as described, for example, in the reference book R.C. Rowe, P.J. Sheskey and P.J. Weller, Handbook of Pharmaceutical Excipients, Sixth Edition, Pharmaceutical Press, 2009. Also, common excipients and procedures for preparing the compositions are described in the book J.P Remington and A. R. Genaro, Remington The Science and Practice of Pharmacy, 20th edition, Lippincott, Williams & Wilkins, Philadelphia, 2000 [ISBN: 0-683-306472] or in the book M.E. Aulton and K.M.G. Taylor, Aulton's Pharmaceutics, the design and manufacture of medicines, 4th edition, Churchill Livingstone Elsevier, 2013 [ISBN: 978-0-7020-4290-4].

The pharmaceutical compositions comprising the glycoconjugate of the invention are preferably for parenteral use.

The compositions typically comprise a vehicle, such as water or a saline solution. Nonaqueous solvents such as ethanol, glycerol or propylene glycol may be added as cosolvents to improve the solubility. The compositions may also comprise preservatives, antioxidants, pH regulating agents, tonicity adjusting agents, suspending agents, or combinations thereof, as are well known in the art.

### Examples

### Example 1: Preparation of the compound of formula (I)

The specific compounds of formula (I) listed in Table 2 were synthetised following the synthetic methods described above, according to Schemes la, Ib and Ic.

**TABLE 2**

| **Example:** | **Structure** |
|---|---|
| 1.1 | Glcα1-3(Fucα1-2)Galβ1-4GlcNAcβ-R |
| 1.2 | Glcα1-3(Fucα1-2)Galβ-R |
| 1.3 | Glcα1-3Galβ-R |
| 1.4 | Glcα1-3(Fucα1-2)Galβ1-3GalNAcβ-R |
| 1.5 | GlcNAcα1-3(Fucα1-2)Galβ1-4GlcNAcβ-R |
| 1.6 | GlcNPropionylα1-3(Fucα1-2)Galβ1-4GlcNAcβ-R |
| 1.7 | GlcNGlycolylα1-3(Fucα1-2)Galβ1-4GlcNAcβ-R |
| 1.8 | GlcNAcα1-3(Fucα1-2)Galβ-R |
| 1.9 | GlcNPropionylα1-3(Fucα1-2)Galβ-R |
| 1.10 | GlcNGlycolylα1-3(Fucα1-2)Galβ-R |
| 1.11 | GlcNAcα1-3Galβ-R |
| 1.12 | GlcNPropionylα1-3Galβ-R |
| 1.13 | GlcNGlycolylα1-3Galβ-R |
| 1.14 | GlcNAcα1-3(Fucα1-2)Galβ1-3GalNAcβ-R |
| 1.15 | GlcNPropionylα1-3(Fucα1-2)Galβ1-3GalNAcβ-R |
| 1.16 | GlcNGlycolylα1-3(Fucα1-2)Galβ1-3GaNAcβ-R |
| R= -O-(CH₂)₃-NH₂ | |

### Example 2: Preparation of glycoconjugates comprising compound of formula (I) with agarose

NHS-activated resin (10 mL) was incubated overnight at 4°C (under mild agitation) with 1.25 mL of 2 mg/mL of the ligands prepared in Example 1 in 1.25 mL of coupling solution (0.2 M NaHCO₃ and 0.5 M NaCl, pH 8.3), and 2.5 mL of 1 mM HCI. Non-reacted groups in the resin were capped for 4h at 4°C with 5 mL of 0.5 M ethanol amine in 0.1 M Tris-CI at pH 8.5.

### Example 3: Preparation of glycoconjugates comprising a compound of formula (I) and magnetic nanoparticles

Glycoconjugates with magnetic nanoparticles were prepared using the same procedure disclosed in Example 2, using NHS-activated magnetic nanoparticles.

### Example 4: Preparation of glycoconjugates comprising a compound of formula (I) and poly-L-lysine

Glycoconjugates of compounds 1 to 16, as disclosed in Example 1, and linear poly-*L*-lysine of a degree of polymerization of 1000 (DP1000), i.e., having 1000 *L*-lysine units in the chain on average, were prepared. The glycoconjugates had a loading degree of about 25% of glycan. The free amino groups on the lysine were capped with glycolic acid. The glycoconjugates prepared can be represented with the following formula: 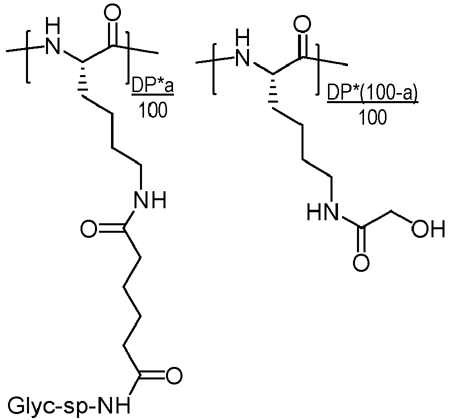 where DP=1000 and a = 25.

### Activation of the ligands with adipic acid succinimidyl diester

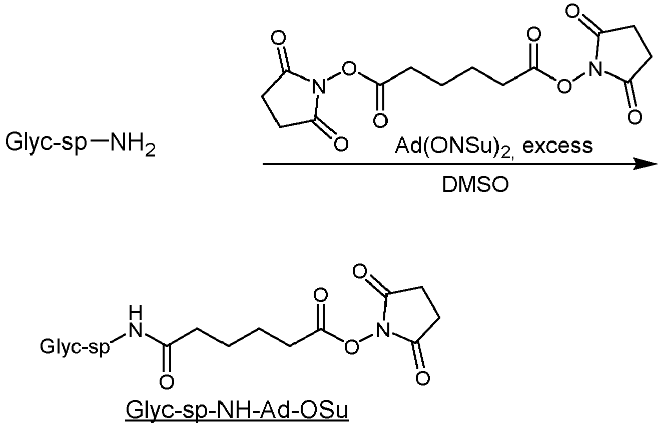

To a stirred solution of adipic acid succinimidyl diester, Ad(ONSu)₂ (452 mg, 1.328 mmol) in DMSO (4 mL) a solution of the compounds of Example 1 (0.166 mmol) in DMSO (1 mL) was added in four portions (~ 0.25 mL) with 10 min interval. The resulting solution was kept for 75 min at room temperature, then acidified with 0.1 mL of AcOH and diluted with 50 ml of cold 1% aqueous AcOH. The precipitate of excessive adipic acid succinimidyl diester was filtered off and washed with 1% aqueous AcOH. The filtrate was evaporated in vacuum (30 °C in a bath) to minimal volume and passed through a Sephadex LH-20 column (180 mL of gel, eluted with 1:1 acetonitrile/water + 1% AcOH). Fractions, containing Glyc-sp-NH-Ad-OSu were combined, evaporated to ~ 2 mL volume, and freeze dried.

### Preparation of glycolic acid acetate succinimide ester (AcOCH₂(CO)ONSu) (capping agent)

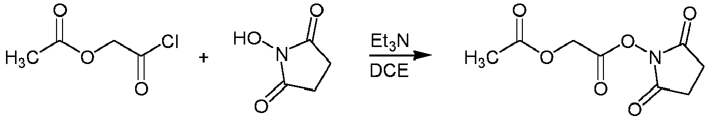

To a stirred cooled (0° C) solution of *N*-hydroxysuccinimide (992 mg, 8.62 mmol) and Et₃N (1.141 mL, 8.21 mmol) in dry 1.2-dichloroetane (15 mL) a solution of acetoxyacetyl chloride AcOCH₂(CO)Cl (1.121 g, 8.21 mmol) in 1.2-dichloroetane (5 mL) was added in several portions. The reaction mixture (precipitate of Et₃N hydrochloride was formed) was stirred at room temperature for 30 min, then AcOH (0.47 mL) was added. The mixture was diluted with 20 mL of 1% AcOH, stirred by shaking, and organic layer was extracted with 20 mL of 1% AcOH one more time. The organic solution was evaporated, and the residue was dried in vacuum. Crystalline material was rubbed with 20 mL of Et₂O/hexane (1:1) mixture, decanted, rubbed with 20 mL of hexane, decanted, and dried in vacuum. Yield of pure AcOCH₂(CO)ONSu was 1394 mg (79%).

TLC: R*_{f}*= 0.67 (chloroform/methanol 10:1 by volume).

¹H NMR (CDCl₃ + 1% d₄-AcOH, 700 MHz, 30°C) δ 7.258 (s, CHCl₃), 4.943 (s, 2H; OCH₂CO), 2.847 (s, 4H; CH₂CH₂ of succinimide), 2.175 (s, 3H; C(O)CH₃) ppm.

### Preparation of the glycoconjugate with poly-L-lysine (a=25)

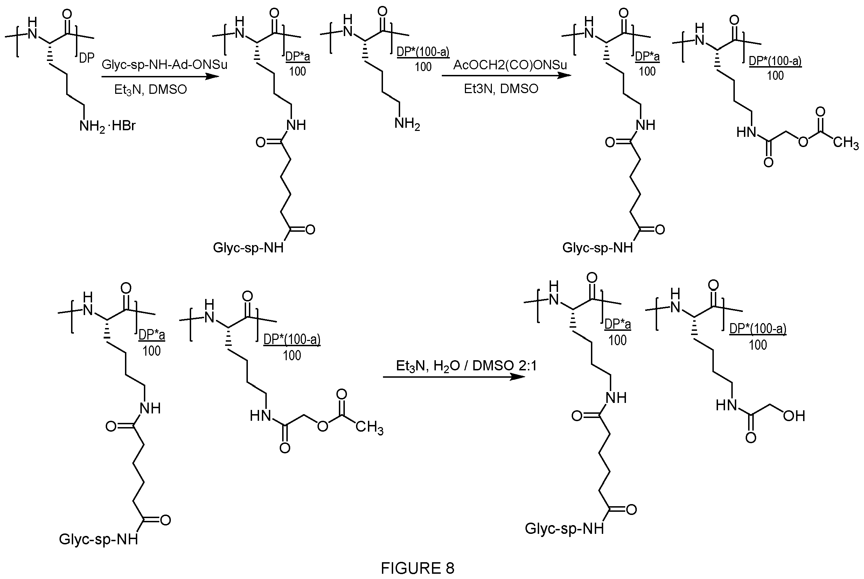

The synthetic scheme is shown in Figure 8. To a stirred solution of polylysine hydrobromide (8.37 mg, 40 micromole of Lys) in DMSO (698 µL, conc. PLys = 12 mg/mL) a solution of Glyc-sp-NH-Ad-ONSu (40*a/100 micromole) in DMSO (conc. Glyc-sp-NH-ONSu = 60 mg/mL) and a solution of Et₃N (3*40*a/100 micromole, 3.336*a µL of 5% by volume in DMSO) were added. The solution was kept for 1 h at room temperature, then dry AcOCH₂(CO)ONSu (2.5*40*(100-a)/100 micromole 0.2152*(100-a) mg) and Et₃N (3*40*(100-a)/100 micromole, 0.139*(120-1.2*a) µL) were added. After 1 hour at room temperature the solution was diluted with two volumes of water and Et₃N was added (2% of total volume). The reaction mixture was kept at room temperature overnight (-16 h), then neutralized with AcOH (equivalent on total Et₃N used). Polylysine conjugate was isolated on Sephadex LH-20 column (100 mL of gel, eluted with 30:70 acetonitrile/water). Fractions contained pure conjugate were evaporated to ~ 2 mL volume and freeze-dried. Yields of Glyc(a%)_PLys conjugates were 92-97%.

The purity and composition of the synthesized glycoconjugates (the percentage of modification of polylysine by Glyc saccharide) was determined by the ¹H NMR spectroscopy by integrating signals of trisaccharide and polylysine fragments.

### Preparation of Glycolyl-poly-L-lysine (DP100) (control)

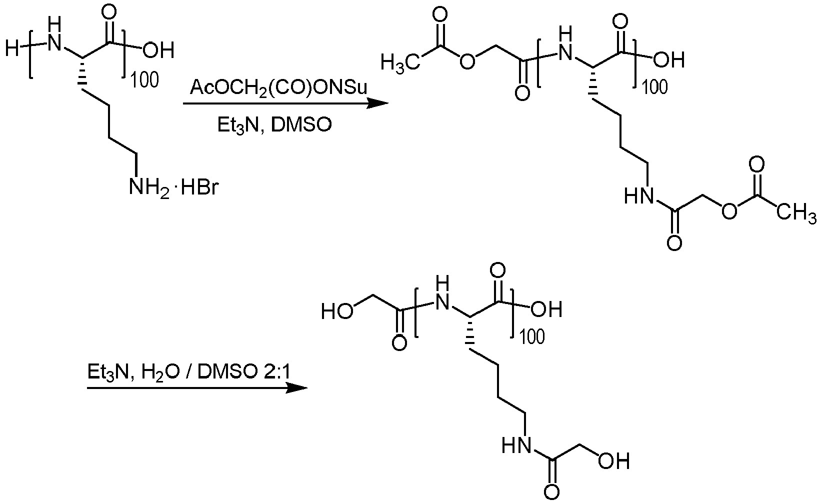

To a stirred solution of polylysine (DP100) hydrobromide (11.43 mg, 54.62 micromole of Lys) in DMSO (750 µL) dry AcOCH₂(CO)ONSu (29.4 mg, 136.5 micromole) and Et₃N (22.8 µL, 164 micromole) were added. After 2 hour at room temperature the solution was diluted with two volumes of water (1500 µL) and Et₃N was added (102 µL, corresponds to -45 µL of excessive free Et₃N, -2% of total volume). The reaction mixture was stirred at room temperature 24 h, the small gel-like precipitate was filtered off and washed with water. Combined filtrates were evaporated to ~ 2 mL volume and polylysine conjugate was isolated on Sephadex LH-20 column (100 mL of gel, eluted with 30:70 acetonitrile/water). Fractions contained pure conjugate were evaporated to ~ 2 mL volume and freeze-dried. Yield of Glycolyl_PLys_DP100 conjugate was 8.6 mg (84%).

¹H NMR of polylysine (DP100) hydrobromide (D₂O, 700 MHz, 30°C) δ 4.750 (s, HOD), 4.365 (dd, 100H, J=8.8 Hz, J=5.7 Hz; α-CH), 3.051 (t, 200H, J=7.6 Hz; ε-CH₂), 1.770 (m, 400H; β-CH₂ and δ-CH₂), 1.474 (m, 200H; γ-CH₂) ppm.

¹H NMR of Glycolyl_PLys_DP100 conjugate (D₂O + TFA, 14 mM/L, 700 MHz, 30°C) δ 4.750 (s, HOD), 4.107 (m, 100H; α-CH), 4.057 (s, 200H; C(O)CH₂O), 3.253 (m, 200H; ε-CH₂), 1.969 (m, 200H; β-CH₂), 1.593 and 1.453 (m, 400H; δ-CH₂ and γ-CH₂) ppm.

### Example 5: Inhibitory capacity of poly-L-lysine glycoconjugate of the invention of anti-A/B antibodies using normal human serum from blood group A or B

The glycopolymer assayed was PLys-DP1000 decorated with the glycans of the invention (prepared in Example 4), or with natural A/B antigens (25% of glycan load).

Maxisorp 96-well plates (Nunc^{®}) were coated overnight at 2-8 °C with 10 µg/ml natural antigen A or B conjugated to polyacrylamide (PAA) in coating buffer (0.05 M carbonate-bicarbonate, pH 9.6). Wells coated only with 10 µg/ml of PAA were considered as the background for each serum sample assessed. After coating, plates were washed three times with phosphate buffered saline (PBS) plus 0.1 % (v/v) Tween20 (Merck), and then blocked during 1 h at 2-8 °C with 0.5 % Tween20 (v/v) in PBS + 1% (w/v) of BSA (Sigma-Aldrich). Serum samples were previously incubated for 15-17 h under controlled mild orbital shaking (183rpm) at 2-8 °C at growing concentrations of glycopolymers (0-1000 µg/ml). Washing steps were repeated and serum samples (1:100 for IgG and IgM; 1:50 for IgA) diluted in 0.1 % Tween20 (v/v) in PBS + 0.3% of BSA (w/v) were added to the wells and incubated during 0.5 h at 25 °C with shaking. After washing, plates were incubated 0.5 h at 25 °C (shaking) with Horseradish Peroxidase (HRP)labeled anti-human IgM, IgA (1:8000) and IgG (1:12000) antibodies diluted in 0.1 % Tween20 (v/v) in PBS + 0.3% of BSA (Invitrogen). After a new washing round, substrate o-phenylenediamine tablet (Sigma-Aldrich) was added to the wells as HRP substrate and incubated 10 min at 25 °C. The reaction was stopped with 3N HCI and the resulting absorbance was registered at 492 nm using a microplate reader (Biotek). For each glycopolymer, the IC₅₀ was calculated considering the inhibition capacity (%) obtained at different polymer concentrations respect to the sample treated only with the vehicle (PBS).

### Example 6: Inhibitory capacity of poly-L-lysine glycoconjugate of the invention of anti-A/B antibodies using pure fractions of polyclonal human anti-A/B antibodies

The glycopolymer assayed was PLys-DP1000 decorated with the glycans of the invention (prepared in Example 4), or with natural A/B antigens (25% of glycan load).

The same method as described in Example 5 was followed, but using pure fractions of human polyclonal anti-A or Anti-B antibodies instead of normal human serum. These fractions were isolated using Sepharose^{®} resins functionalized with natural A/ B antigens.

### Example 7: Removal efficacy of agarose and MNP glycoconjugates of the invention of anti-A/B antibodies from normal human serum

The glycopolymers assayed were Sepharose^{®} functionalized with the glycans of the invention (prepared in Example 2) and magnetic nanoparticles functionalized with the glycans of the invention (prepared in Example 3), and were compared to resins and MNPs functionalized the natural A/B antigens.

The anti A/B antibodies in the normal human serum tested were quantified by ELISA and printed glycan array technology before (baseline) and after passing the serum through the functionalized resin or the functionalized MNPs.

### Example 8: Purification of human polyclonal anti-A/B antibodies with agarose and MNP glycoconjugates of the invention

The objective of this assay was to purify human polyclonal anti A/B antibodies for inhibition assays.

Commercial NHS-Activated Sepharose and MNP were functionalized with the glycans of the invention according to manufacturer instructions. NHS-activated resin (5-10 mL) and MNP (0,5-1mL) was incubated overnight at 4°C (under mild agitation) with 1.25 mL of 2 mg/mL of the ligands prepared in Example 1 in 1.25 mL of coupling solution (0.2 M NaHCO₃ and 0.5 M NaCl, pH 8.3), and 2.5 mL of 1 mM HCI. Non-reacted groups in the support were capped for 4h at 4°C with 5 mL of 0.5 M ethanol amine in 0.1 M Tris-CI at pH 8.5. After washing steps (PBS-Tween 0,1%, 0,5 M NaCl) the supports were blocked with PBS-Tween 0,01% and BSA 1% (w/v). The blocked supports were incubated (in batch) with pooled human serum (25% v/v, in PBS) of Group A and Group B (gentle shaking, 4-8°C, 2h). Then, in the case of Sepharose, the resin plus serum was introduced in a column (20X2 cm) to separate the unbound serum fraction. For MNP, unbound serum fraction was separated using magnetic separators. After a new round of washing steps, the bound antibodies (anti-A and Anti-B) were eluted from the support with Glycine-HCI Buffer (0.1 M, pH 3.0). Eluted antibodies fractions were immediately neutralized to a pH around 7 and quantified by Bradford (1976). Purity was assessed by SDS-PAGE.

## Claims

1. Compound of formula (I): 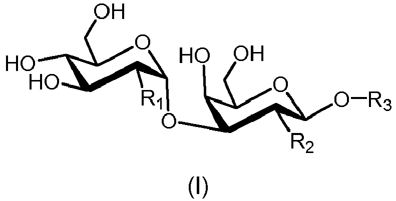 wherein:
R₁ is selected from OH and -NH-CO-R₅, wherein R₅ is C₁-C₃ alkyl and wherein one or more hydrogen atoms in R₅ may be replaced by a hydroxyl group;
R₂ is selected from OH and a group of formula (II): 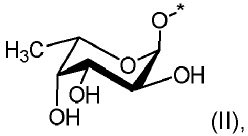 wherein * indicates the point of attachment of said group with the rest of the molecule;
R₃ is selected from R₄-Z, a group of formula (III) and a group of formula (IV): 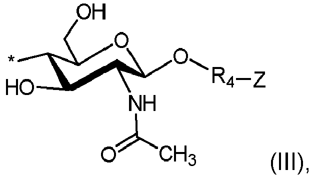 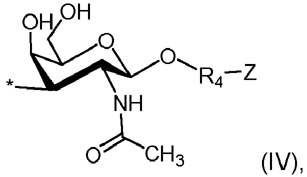 wherein
* indicates the point of attachment of said group with the rest of the molecule, R₄ is a spacer group, and
Z is selected from -NH₂, -COOH, and -SH.

2. The compound according to claims 1 or 2, **characterized in that** R₁ is selected from OH, N-acetyl, N-propionyl and N-glycolyl.

3. The compound according to claims 1 or 2, **characterized in that** when R₂ is OH, then R₃ is R₄-Z.

4. The compound according to claim 1, **characterized in that** the compound of formula (I) is selected from:
1) Glcα1-3(Fucα1-2)Galβ1-4GlcNAcβ-R
2) Glcα1-3(Fucα1-2)Galβ-R
3) Glcα1-3Galβ-R
4) Glcα1-3(Fucα1-2)Galβ1-3GalNAcβ-R
5) GlcNAcα1-3(Fucα1-2)Galβ1-4GlcNAcβ-R
6) GlcNPropionylα1-3(Fucα1-2)Galβ1-4GlcNAcβ-R
7) GlcNGlycolylα1-3(Fucα1-2)Galβ1-4GlcNAcβ-R
8) GlcNAcα1-3(Fucα1-2)Galβ-R
9) GlcNPropionylα1-3(Fucα1-2)Galβ-R
10) GlcNGlycolylα1-3(Fucα1-2)Galβ-R
11) GlcNAcα1-3Galβ-R
12) GlcNPropionylα1-3Galβ-R
13) GlcNGlycolylα1-3Galβ-R
14) GlcNAcα1-3(Fucα1-2)Galβ1-3GalNAcβ-R
15) GlcNPropionylα1-3(Fucα1-2)Galβ1-3GalNAcβ-R
16) GlcNGlycolylα1-3(Fucα1-2)Galβ1-3GalNAcβ-R
wherein R= -O-R₄-Z.

5. A process for the preparation of the compound of claim 1, **characterized in that** it comprises the following steps:
a) Preparing a compound of formula **C** from a compound of formula **A** and a compound of formula **B**: 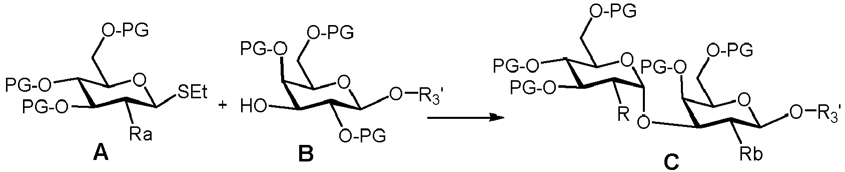 wherein
PG are protecting groups;
Rₐ is azide or O-PG;
R₃' is selected from R₄-Z', a group of formula (III) and a group of formula (IV), wherein the group of formula (III) and the group of formula (IV) are as defined in claim 1, but wherein the OH groups in the carbohydrate moiety in formulas (III) and (IV) are protected, and wherein Z' is a group of formula Z as defined in claim 1 which is protected;
R_{b} is OH or O-PG; and
b)
b1) when R_{b}=OH, linking a protected fucose residue through a α(1→2) glycosidic linkage at this position to provide, after deprotection, the compound of formula (I) wherein R₂ is a group of formula II;
b2) when R_{b}=O-PG, deprotecting the compound of formula C to provide a compound of formula (I) wherein R₂ is OH;

6. A glycoconjugate comprising the compound of formula (I) as defined in anyone of claims 1 to 4 and polymeric backbone.

7. The glycoconjugate of claim 6, **characterized in that** the polymer is selected from homologous and heterologous α-amino acid polymers (homo and heteropolypeptides), proteins, lipids, nucleic acids, acrylic acid polymers, methacrylic acid polymers, acrylic and methacrylic acid copolymers, polysaccharides such as chitosan or agarose, polyethylene, polypropylene, polystyrene, polyvinyl butyrate, poly (vinyl chloride) and dendrimers, preferably the polymer is selected from polyacrylic acid, a polysaccharide resin and an α-aminoacid homopolymer, and more preferably the polymer is selected from polyacrylic acid, agarose and polylysine.

8. The glycoconjugate of claim 7, **characterized in that** it is a polyacrylamide of the following formula: 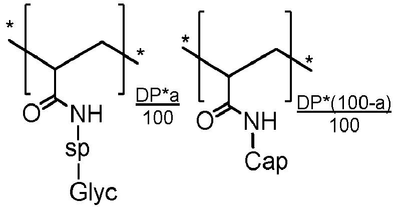 wherein
"Cap" means a capping agent,
"DP" is the degree of polymerization of the polymer,
"a" is the load percentage of the carbohydrate in the glycoconjugate,
"sp" represents the spacer R₄, as defined in claim 1, and
"Glyc" represents anyone of the groups: 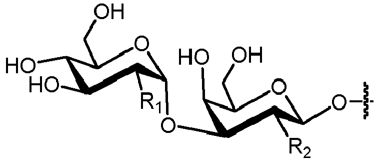 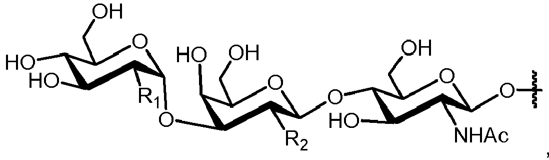 or 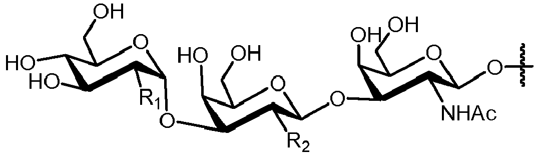 wherein R₁ and R₂ are as defined in anyone of claims 1 to 3;
and preferably Glyc is the carbohydrate moiety of the compounds of claim 4.

9. The glycoconjugate of claim 7, **characterized in that** it comprises a compound of formula (I) linked to agarose as polymeric backbone.

10. The glycoconjugate of claim 7, **characterized in that** it is a poly-*L*-lysine glycoconjugate of the following formula: 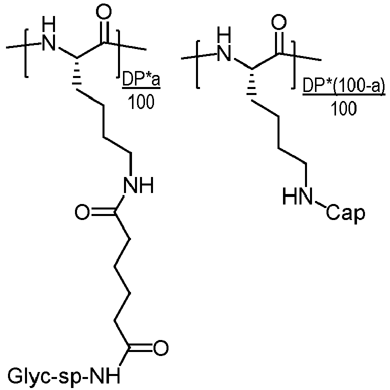 wherein
"Cap" means a capping agent;
"DP" is the degree of polymerization of the polymer, which is preferably comprised between 50 and 2000, more preferably between 100 and 1000;
"a" is the load percentage of the carbohydrate in the glycoconjugate, which is comprised between 2 and 90, preferably comprised between 5 and 50;
"sp" represents the spacer R₄, as defined in claim 1, and
"Glyc" is a carbohydrate moiety as defined in claim 8.

11. The glycoconjugate of claim 7, **characterized in that** it comprises a compound of formula (I) linked to a magnetic nanoparticle.

12. Use of the glycoconjugate according to anyone of claims 6 to 11 for inhibiting and/or removing anti-A and/or anti-B antibodies from samples of blood or other blood derivatives and by-products.

13. Glycoconjugate according to claim 6 for use in medicine, preferably, for use in the treatment and/or prevention of rejection or haemolytic reactions in subjects undergoing organ transplantation or blood transfusion, respectively, from incompatible donors.

14. Glycoconjugate for use according to claim 13, wherein the glycoconjugate comprises poly-*L*-lysine as polymeric support and is preferably the glycoconjugate of claim 10.

15. Pharmaceutical composition comprising the glycoconjugate of claim 10 and at least one pharmaceutically acceptable excipient.
